# EUROPEAN PATENT APPLICATION

(11) **EP 2 508 519 A1**
(43) Date of publication of application: **10.10.2012**
(21) Application number: 11161489.7
(22) Date of filing: 07.04.2011
(51) Int. Cl.: C07D 333/20

(54) **"Process for the preparation of duloxetine and its hydrochloride salt"**

(71) Applicant: Bioindustria Laboratorio Italiano Medicinali S.p.A In forma abbreviata Bioindustria L.I.M. S.p.A., 15067 Novi Ligure (AL) (IT)
(72) Inventor: Moro, Alberto, 15067, NOVI LIGURE (AL) (IT); Tarditi, Roberto, 15067, NOVI LIGURE (AL) (IT); Giordani, Cristiana, 15067, NOVI LIGURE (AL) (IT); Leccese, Maria Laura, 15067, NOVI LIGURE (AL) (IT); Mantelli, Cristina, 15067, NOVI LIGURE (AL) (IT)
(74) Representative: Minoja, Fabrizio

(57) **Abstract**

A process for the preparation of duloxetine (N-methyl-3-(1-naphthyloxy)-3-(thiophen-2-yl)-propanamine) of formula IV: 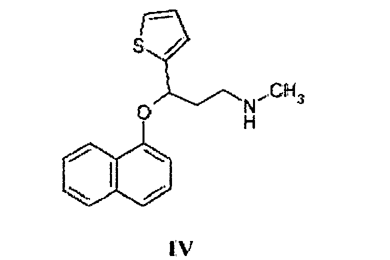 and its hydrochloride salt, comprising the following steps:
Compound (II) is reacted with an alkali metal hydride in sulfolane to (III).
Crude (III) is purified by a double extraction process;
Purified (III) is then demethylated to give (IV).
The process is characterized by being safer, allowing solvent recovery limiting the racemization of the key intermediates.

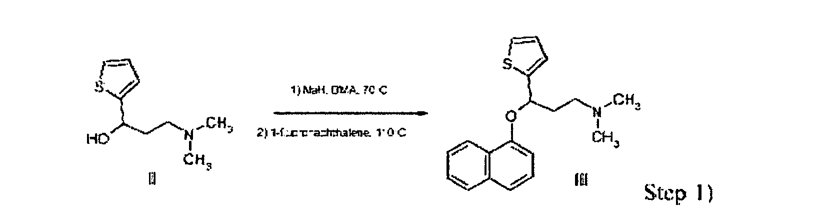

## Description

### Field of the invention

The present invention relates to an improved process for the preparation of duloxetine and its hydrochloride salt.

### Background of the invention

Duloxetine hydrochloride is the compound represented in formula (**I**)

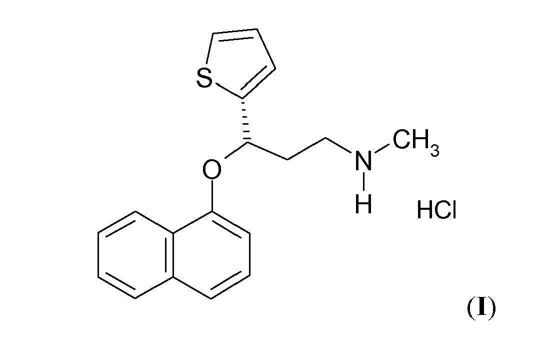

It is the common name of (+)-(S)-N-methyl-3-(1-naphthyloxy)-3-(thiophen-2-yl)-propanamine hydrochloride, which is an active pharmaceutical ingredient marketed with the trade name of Cymbalta™and Yentreve™. The compound is effective as an anti-depressant agent.

Racemic duloxetine was firstly described in EP273658 to Lilly. The synthetic process disclosed in this patent for the preparation of the compound, as the free base in racemic form **IV,** is depicted in Scheme.

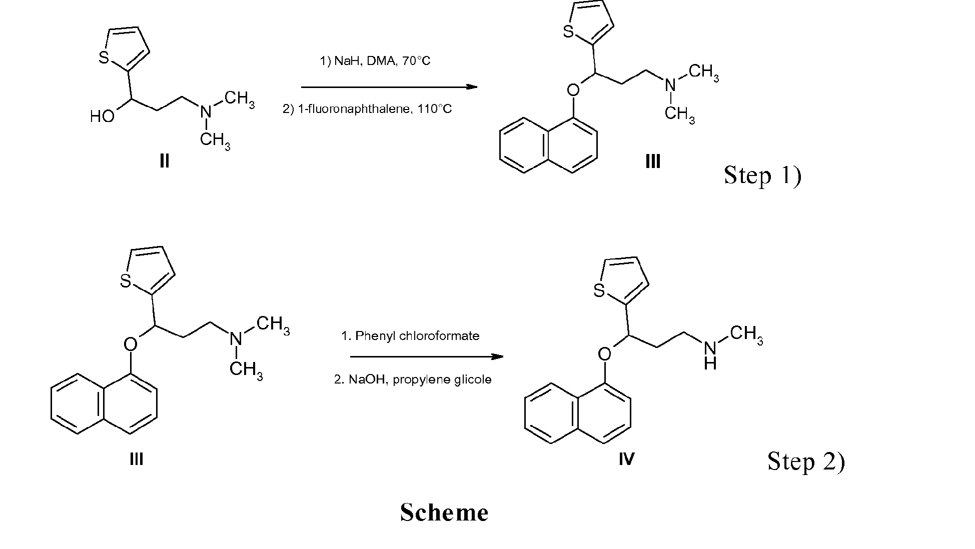

In step 1) treatment of racemic alcohol **II** with sodium hydride in N,N-dimethylacetamide (DMA) at 70°C generates the oxygen anion of **II** which subsequently reacts with 1-fluoronaphthalene at 110°C to provide racemic N-methyl-duloxetine **III**. Demethylation of **III** to provide the racemic free base **IV** is then performed in step 2) by reaction of **III** with phenylchloroformate followed by basic treatment.

Many other patent documents describe similar processes, which can be conduced both on racemic or enantiopure alcohol **II,** wherein in step 1 **II** is reacted with sodium hydride or other bases in polar solvents and then it is condensed with 1-fluoronaphtalene in order to obtain **III.**

For example, EP 0650965 B1 discloses a process for the preparation of the (S)-(+) enantiomer of intermediate **III**, comprising reacting the (S)-(-) enantiomer of **II** with sodium hydride, potassium benzoate or potassium acetate, and 1-fluoronaphthalene in an organic solvent, which is preferably DMSO.

WO2004/056795 A1 discloses a process for the preparation of racemic or chiral (S)-(+) duloxetine by O-alkylation of racemic **II** or its (S)-(-) enantiomer with 1-fluoronaphthalene in potassium hydroxide/DMSO under phase transfer conditions, followed by demethylation of racemic of chiral **III** obtained.

WO2007/096707 A2 discloses a method for preparing (S)-duloxetine and its intermediates from the (S)-(-) enantiomer of compound **II** and 1-fluoronaphthalene and using an alkaline metal hydroxide or alkoxide in DMSO or DMSO-cosolvent mixtures, in the absence of a phase-transfer catalyst.

WO2007077580 A2 discloses a process for the preparation of (S)-duloxetine oxalate substantially free from its (R)-enantiomer by reaction of the (S)-(-) enantiomer of compound **II** and 1-fluoronaphthalene in a NaOH/DMSO mixture in the presence of tert-butylammonium bromide, followed by demethylation with phenylchloroformate and formation of the oxalate salt. The process is performed without isolation of intermediates. Duloxetine oxalate may then be converted in duloxetine hydrochloride.

WO2008/107911 A2 discloses a process for the preparation of duloxetine hydrochloride wherein the first step is the reaction of racemic intermediate **II** with 1-fluoronaphthalene in the presenza of a base selected from sodium amide, potassium amide or potassium bis(trimethylsilyl)amide, in an apolar aprotic solvent selected from DMSO, sulfolane, N,N-dimethylformamide (DMF), N,N-dimethylacetamide (DMA), N-methylpirrolidone (NMP), to provide racemic **III**.

WO/2007/095200 A2 discloses a process for preparing the (S)-(+) enantiomer of **III** comprising reacting the S-(-) enantiomer of **II** and 1-fluoronaphthalene or 1-chloronaphthalene in the presence of a base such as an alkali metal hydroxide or sodium or lithium alkoxides. The reaction is performed in a polar aprotic solvent selected from the group comprising aromatic hydrocarbons, ionic liquid, DMSO, DMF, DMA, acetonitrile, sulfolane, nitromethane, propylene carbonate, in the absence of a phase transfer catalyst.

The above reactions for the preparation of intermediate **III** are generally described to be conduced in solvents such as DMSO, which is known to form with sodium hydride or other bases the desmyl anion, which is dangerous if heated over 50°C and has been reported to produce explosions. Other solvents employed, such as DMF or DMA have also been reported to be dangerous when heated with sodium hydride (C&EN Sept 13, 1982 p.42-43; Sax & Lewis, Dangerous properties of Materials).

Moreover the solvents employed in this step are very soluble in water and in organic solvents and they need long and tedious work-ups in order to isolate compound **III** essentially free of residues of these solvents. This commonly consists in use of mixture of solvents for the extraction (e.g., hexane/ethyl acetate) or repeated washings with water of the organic phase. Thus, solvents used for these kind of extractions cannot be easily recycled and also aqueous phases contaminated with DMSO or DMF or DMA can not easily be disposed of in a waste water treatment plant and should be sent for incineration, with a consequent increase in costs of production and disposal.

In some more recent patent applications (EP 1171417), the solvent used for this transformation is NMP. Anyway, when the reaction is performed in this solvent it proceeds with a consistent degree of racemisation (S/R ratio about 60:40) if starting from chiral intermediate **II** and this requires further separation of the unwanted enantiomer.

In any case, the common purification described for compound **III** is the transformation in its oxalate salt. This adds three further steps to the synthetic route: salt formation, crystallization of the oxalate and basification of the oxalate salt to obtain **III** as the free base. Therefore it is not desirable on industrial scale.

Most of the concerns about the safe use of sodium hydride and DMSO and on racemization problems are reported also in US2007/0167636, where the reaction to obtain compound **III** is performed in glymes (preferably diglyme) or Toluene/DMSO mixture. In this case compound **III** is isolated in the form of its phosphate salt.

It may be thus desirable to find a new synthetic process which allows to obtain duloxetine molecule in high yield, with an easier purification method and la ow degree of racemisation in order to improve the industrial application of the synthesis.

### Summary of the invention

The inventors have surprisingly found that the reaction described in step 1) of Scheme can be conduced in a polar aprotic solvent such as sulfolane, which is not reactive towards sodium hydride. The work-up of this reaction is made using water for the quenching and cyclohexane as solvent for the extraction of the product **III**. As sulfolane is very poorly soluble in cyclohexane, the majority of sulfolane remains in the aqueous phase while cyclohexane efficiently extracts the desired product.

A further extraction step is then made, at controlled pH values, of the residue obtained from the cyclohexane extraction, in order to separate **III** from the unreacted starting materials **II** and 1-fluoronaphthalene. This allows to obtain the desired product **III** with chromatographic purity higher than 98% and an assay higher than 98% as well, without precipitating **III** as a salt.

In this way the productivity and the overall yield of the synthesis are increased, avoiding the salt formation and basification steps for purifying **III** and allowing the distillation and recovery of the cyclohexane used in the extraction in order to reuse it.

Also mother liquors containing sulfolane can be disposed of in a waste water treatment plant reducing both costs for disposal and environmental impact of the process comparing to the incineration.

A second feature of the present invention is that the reaction for obtaining **III** proceeds in sulfolane with a limited racemisation when the starting alcohol **II** is chiral, thus allowing to obtain **III** with a S/R ratio of at least 95/5 starting from (S)-alcohol **II**, whereas using DMA or NMP the ratio is of 83/17 or 60/40, respectively.

Another feature of the present invention is that compound **III** can directly be subjected to the following step of demethylation without isolation. The only isolated product will be directly the free base **IV**. This allows a rapid synthetic process which can be conduced with less time and increasing productivity.

### Detailed description of the invention

The present invention relates to a process for obtaining the compound N-methyl-3-(1-naphthyloxy)-3-(thiophen-2-yl)-propanamine **IV**, in particular the compound (+)-(S)-N-methyl-3-(1-naphthyloxy)-3-(thiophen-2-yl)-propanamine (duloxetine base) and its hydrochloride salt of formula **I** (duloxetine hydrochloride), comprising the following steps:
a) contacting in sulfolane N,N-dimethyl-3-hydroxy-3-(2-thienyl)propanamine of formula **II** 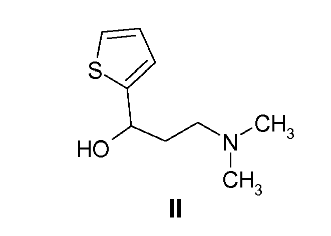 and an alkali metal hydride, at a temperature and for a time sufficient to generate the alkali metal salt of N,N-dimethyl-3-hydroxy-3-(2-thienyl)propanamine **II**, to provide a sulfolane solution containing the alkali metal salt of N,N-dimethyl-3-hydroxy-3-(2-thienyl)propanamine **II**;
b) contacting the sulfolane solution obtained in Step a) with 1-fluoronaphthalene at a temperature and for a time sufficient to provide a sulfolane solution containing N,N-dimethyl-3-(1-naphthyloxy)-3-(thiophen-2-yl)-propanamine **III**; 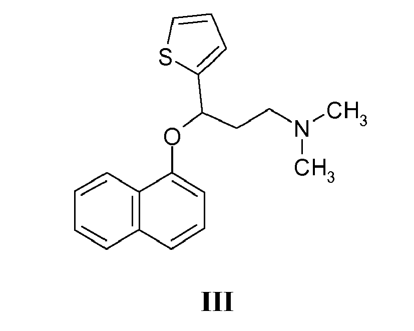
c) adding water to the sulfolane solution obtained in Step b) and extracting the obtained mixture with cyclohexane, to provide an aqueous phase containing sulfolane and a cyclohexane solution containing compound **III**;
d) evaporating the cyclohexane solution obtained in Step c) to provide a residue containing compound **III**;
e) partitioning the residue obtained in Step d) between an acidic aqueous solution and hexane, to provide an acidic aqueous phase containing **III**;
f) neutralizing the acidic aqueous phase containing **III** obtained in Step e) with a base and extracting with toluene, to provide a toluene solution containing compound **III**;
g) subjecting the toluene solution obtained in Step f) to N-demethylating conditions, to obtain compound N-methyl-3-(1-naphthyloxy)-3-(thiophen-2-yl)-propanamine **IV** 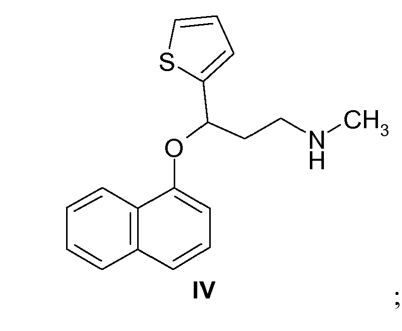 and
h) optionally converting compound **IV** obtained in Step g) into its hydrochloride salt.

In a preferred embodiment the compound of formula **II** is (S)-(-)-N,N-dimethyl-3-hydroxy-3-(2-thienyl)propanamine, the compound of formula **III** is (S)-(+)-N,N-Dimethyl-3-(1-naphthalenyloxy)-3-(2-thienyl)propanamine (N-methyl duloxetine) and the compound of formula **IV** is (S)-(+)-N-methyl-3-(1-naphthyloxy)-3-(thiophen-2-yl)-propanamine (duloxetine base), which may then be converted in its hydrochloride salt of formula **I** (duloxetine hydrochloride).

Step a) of the process according to the invention is performed suspending an alkali metal hydride, preferably sodium hydride, in sulfolane anhydrous at a temperature between 40 to 80°C, most preferably at 70°C, adding to this suspension a solution of **II** in sulfolane anhydrous, heating the resulting solution at a temperature between 40 to 80°C for a time of 30 minutes to 2 hours. The most preferred time of formation of the salt of compound **II** is of 1h at a temperature of 70°C.

In Step b) 1-fluoronaphthalene is added to the solution of the alkali metal salt of compound **II** obtained in Step a) and the resulting solution is further stirred for 1 to 5 hours at a temperature between 40 to 130°C, but preferably for 3 hours at 110°C.

In Step c), extraction of the reaction mixture containing **III** is made using cyclohexane after quenching with water. This allows to extract only **III** together with the free base of **II** and 1-fluoronaphthalene, leaving sulfolane in the aqueous phase. The aqueous phase containing sulfolane can be disposed of to a waste water treatment plant, whereas cyclohexane can be recovered in Step d) by distillation *in vacuo.*

In Step e) the oil obtained in Step d) from the evaporation of cyclohexane is purified by dissolution in an acidic solution, preferably in a diluted acetic acid solution at pH between about 4.0 and about 6.0 and preferably at about pH 4.5, and washing the acidic solution with hexane in order to remove 1-fluoronaphthalene. This leaves compound **III** in the aqueous phase where it can be recovered following neutralisation with a base and extraction with an organic solvent.

Accordingly, in Step f) the aqueous phase obtained after the extractions with hexane in Step e) is brought to a pH value between about 6.5 and about 7.5, most preferably at about 7.0, with a base, preferably with ammonium hydroxide, and the desired compound **III** is extracted with toluene, to provide a toluene solution containing **III**.

Compound **III** obtained in this manner is substantially pure (HPLC purity higher than 98%) and can be used directly in the following step without further purification.

Accordingly, in Step g) the toluene solution containing **III** obtained in Step f) is directly subjected to the N-demethylation step to provide **IV**. Demethylation can be accomplished in a one-pot reaction, for example by addition of phenylchloroformate and diisopropylethylamine (DIPEA) to the toluene solution containing **III**, followed by basic hydrolysis of the intermediate carbamate, without isolation of the latter, as disclosed in EP1730132.

Compound **IV** obtained as free base according to the process of the invention may then be converted to the corresponding hydrochloride salt with methods known to those skilled in the art. In particular, when the compound of formula **IV** is Duloxetine base it can be converted into Duloxetine hydrochloride **I** following the procedures described in WO 2006/071868 or WO2006/126213 or US2007/0238883.

The process of the invention is particularly suitable for industrial use and presents many ecological advantages such as the possibility of recycling all the solvents involved in the process (cyclohexane, hexane and toluene) apart from sulfolane, which anyway can be easily disposed of by a waste water treatment plant.

Also unreacted starting materials such as **II** or 1-fluoronaphthalene can be easily recovered form the organic and aqueous phase increasing the overall yield of the process.

The following are the advantages of the process of the invention over the processes known in the art.
1) The combined use of sulfolane and sodium hydride allows to run safely the first step of the synthesis of duloxetine, i.e. the conversion of **II** to **III**. As discussed above, all of the processes that use sodium hydride in the presence of DMSO, DMF or DMA may lead to explosion of the reaction mixture. The mixture of DMF and sodium hydride is reported in the Sax & Lewis 's Dangerous Properties of Industrial Materials to give a violent reaction with ignition above 50°C. Buckey, J. et al., Chem. Eng. News, 1982, 60(28),5 describes the thermal runaway of a pilot plant reactor containing sodium hydride and dimethylformamide from 50°C. Accelerating Rate Calorimetry (ARC) tests showed exothermic activity at a temperature as low as 26°C. Similar behaviour was also seen with DMA. De Wall, G., Chem. Eng. News, 1982, 60(37), 5 reports a similar incident, wherein runaway started at 40°C, and rose to 100°C in less than 10 minutes, boiling off most of the DMF.

The mixture of sodium hydride (and also sodium amide used in example 1 of WO2008107911 A2) with DMSO generates the dimsyl anione. Although very often used in the lab scale, this anion is unstable and it is used on a large scale only with many precautions. In a technical bulletin Gaylord Chemicals, a producer of DMSO states that they have been able to produce this anion up to a 22-liter scale and that chemists wishing to use it must carefully read the sections of the bulletin dealing with stability, general handling and safety considerations (The Dimethyl Sulfoxide (DMSO) Anion - Dimsyl Ion, Gaylord Chemicals, Bulletin #110, October 2007, available online at http://www.gaylordchemical.com/bulletins/bulletin110b/Bulletin110B.pdf; see page 2).

The above findings have been confirmed by studies performed by the inventors of the present application on the reactivity of sodium hydride in various solvents using Solid State Calorimetry (SSC), wherein only a low exothermic effect was observed mixing anhydrous sulfolane and sodium hydride. In the subsequent gradual heating of the mixture other exothermic effects were not observed, which on the other hand occurred with the mixtures of DMA or NMP with sodium hydride.
2) Another important advantage of the process of the invention is the very low level of racemisation by which chiral intermediate **III** is obtained starting from chiral intermediate **II** using sodium hydride in sulfolane, contrary to what is observed performing the same reaction in DMA e NMP. In many of the process disclosed in the patents in which sodium hydride is used (for example EP0650965B1 and WO2004/056795), it is mandatory to add catalysts in order to avoid or minimise racemisation.
3) The extraction with cyclohexane of the reaction mixture obtained after quenching with water, (Step c)), leaves sulfolane in the aqueous phase. Otherwise, later separation of this solvent from the reaction product would be difficult, given that sulfolane is miscible with many organic solvents.
4) The subsequent extractions at controlled pH, first at pH about 4.5 with hexane (to remove 1-fluoro-naphthalene) and then at pH about 7 with toluene (to remove other starting material **(II)** allow to efficiently purify compound **III**, avoiding transformation of the latter in its oxalate salt and thus significantly increasing the efficiency of the process.
5) The recovered raw materials can be re-introduced in the process.
6) The obtainment of a toluenic solution of compound **III** allows to perform directly on this solution the subsequent demethylation step to provide compound **IV**, without the need of any further work-up.

### Examples

### Example 1

### Preparation of (S)-(+)-N,N-dimethyl-3-(1-naphthalenyloxy)-3-(2-thienyl)propanamine III

In a 5 liters four necked round bottom flask equipped with mechanical stirrer, reflux condenser and thermometer sulfolane (300 mL) and sodium hydride 60% in mineral oil (25 g) are loaded. To the resulting suspension a solution of **II** [(S)-(-)-N,N-dimethyl-3-hydroxy-3-(2-thienyl)propanamine] (100 g, in 450 ml of sulfolane) is quickly added.

The suspension is heated to 70°C for 1 hour, 1-fluoronaphthalene (77 mL) is added and the mixture is further heated at 110°C for 3 hours.

The mixture is then cooled at 10°C and quenched adding 3500 mL of water.

Cyclohexane is then added (1L) and the phases are separated.

The aqueous phase is further extracted with cyclohexane, the combined organic layer is washed with water and evaporated to dryness.

The distilled cyclohexane can be reused in the same step, wile the resulting oil is dissolved in a 20% aqueous solution of acetic acid at pH 4.5 and extracted with hexane twice.

The hexane phase can be evaporated to residue to obtain distilled hexane which can be reused and a residue of 1-fluoronaphthalene which is recovered.

The aqueous phase is made neutral with ammonium hydroxide and extracted with toluene (1L).

About 100 mL of toluene are distilled in order to eliminate water as a water/toluene azeotrope and the resulting toluene solution containing **III** is used in the following step.

### Example 2

### Preparation of (S)-duloxetine base

To the toluene solution obtained in Example 1, N,N-diisopropylethylamine (95 mL) and phenyl chloroformate (65 mL) are added. The mixture is heated at 50°C for 2 hours then cooled to room temperature. The toluene solution is washed with 700 mL of diluted sodium bicarbonate solution, with diluted hydrochloric acid solution and with water.

Then KOH solid is added (100g) and the suspension is heated to reflux for 1 hour.

The suspension is cooled, filtered and the organic phase is washed with water to neutrality.

The organic phase is evaporated to dryness to obtain (S)-duloxetine base as an oil (96 g; 60% yield from **II**).

From the compound obtained in Example 2, duloxetine hydrochloride having specifications needed for the use as an API, for example those described in Pharmeuropa vol.22 n.4, pag 492-494, can then be easily prepared by a person skilled in the art, for example following the procedures described in example 4 of WO2006/126213 or in example 15 of US2007/0238883.

## Claims

1. A process for the preparation of N-methyl-3-(1-naphthyloxy)-3-(thiophen-2-yl)-propanamine of formula **IV**: 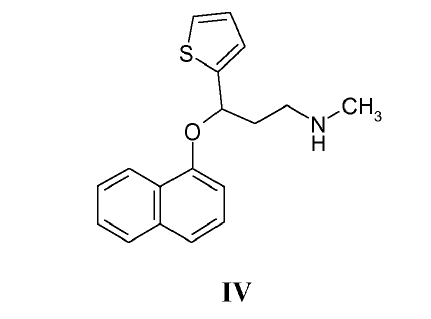 comprising the following steps:
a) contacting in sulfolane N,N-dimethyl-3-hydroxy-3-(2-thienyl)propanamine of formula **II**: 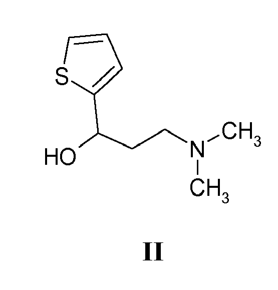 and an alkali metal hydride, at a temperature and for a time sufficient to generate the alkali metal salt of N,N-dimethyl-3-hydroxy-3-(2-thienyl)propanamine **II**, to provide a sulfolane solution containing the alkali metal salt of N,N-dimethyl-3-hydroxy-3-(2-thienyl)propanamine **II**;
b) contacting the sulfolane solution obtained in Step a) with 1-fluoronaphthalene at a temperature and for a time sufficient to provide a sulfolane solution containing N,N-dimethyl-3-(1-naphthyloxy)-3-(thiophen-2-yl)-propanamine **III**; 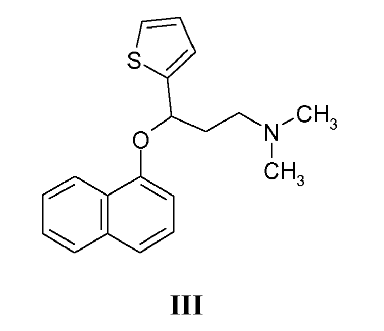
c) adding water to the sulfolane solution obtained in Step b) and extracting the obtained mixture with cyclohexane, to provide an aqueous phase containing sulfolane and a cyclohexane solution containing compound **III**;
d) evaporating the cyclohexane solution obtained in Step c) to provide a residue containing compound **III**;
e) partitioning the residue obtained in Step d) between an acidic aqueous solution and hexane, to provide an acidic aqueous phase containing **III**;
f) neutralizing the acidic aqueous phase containing **III** obtained in Step e) with a base and extracting with toluene, to provide a toluene solution containing compound **III**;
g) subjecting the toluene solution obtained in Step f) to N-demethylating conditions, to obtain compound N-methyl-3-(1-naphthyloxy)-3-(thiophen-2-yl)-propanamine **IV**.

2. The process of claim 1 in which in Step a) the alkali metal salt hydride is sodium hydride.

3. The process of claim 1 in which the compound of formula **II** is (S)-(-)-N,N-dimethyl-3-hydroxy-3-(2-thienyl)propanamine, the compound of formula **III** is (S)-(+)-N,N-dimethyl-3-(1-naphthalenyloxy)-3-(2-thienyl)propanamine and the compound of formula **IV** is (S)-(+)-N-methyl-3-(1-naphthyloxy)-3-(thiophen-2-yl)-propanamine.

4. The process according to claim 1 wherein the compound of formula IV is (S)-(+)-N-methyl-3-(1-naphthyloxy)-3-(thiophen-2-yl)-propanamine (duloxetine base).

5. The process of claim 1, further comprising the conversion of compound **IV** obtained in Step g) into its hydrochloride salt.

6. The process of claims 1 and 5, wherein the hydrochloride salt is duloxetine hydrochloride.
